# EUROPEAN PATENT SPECIFICATION

(11) **EP 0 846 445 B1**
(45) Date of publication and mention of the grant of the patent: **21.07.2004**
(21) Application number: 96119270.5
(22) Date of filing: 02.12.1996
(51) Int. Cl.: A61B 19/02, A61L 2/26, B65D 75/30

(54) **Sealable sterilizable bag**
Verschliessbarer sterilisierbarer Beutel
Sachet stérilisable scellable

(43) Date of publication of application: 10.06.1998
(73) Proprietor: Advanced Technology Materials, Inc., Danbury, CT 06810-4169 (US)
(72) Inventor: Huynen, Marc, 1370 Jodoigne (BE); Huynen, Stéphane, 3360 Bierbeek (BE)
(74) Representative: Bird, Ariane

(56) References cited:
- US-A- 3 685 720
- US-A- 3 754 700
- US-A- 3 768 725
- US-A- 3 819 106
- US-A- 4 121 714
- US-A- 4 203 520
- US-A- 4 270 658
- US-A- 5 222 600
- US-A- 5 308 691

## Description

### Background of the Invention

The present invention relates to a sealable sterilizable bag or pouch which is suitable for introduction of an object followed by the bag being sealed and sterilized using steam at at least 125 °C as well as being suitable for other sterilization methods such as gamma radiation and ethylene oxide sterilization.

US-4,121,714 describes a sterilizable pouch which may be sealed and then opened by cutting with scissors. The pouch has a layer of paper on one side and a layer of heat sealable polymeric material on the other.

US-4,367,816 describes a sterilizable pouch including a pealable opening for removal of the stored object. Pealable seals are weaker than secure seals and may not be suitable for steam sterilization at 125 °C and above. Hence, such bags have been limited to gamma radiation and ethylene oxide gas sterilization.

US-3,754,700 and US-3,768,725 describe bags suitable for gas sterilization. US-3,819,106 describes a sample bag that has an access opening over which a closure membrane can be attached by means of an adhesive coating.

It is an object of the present invention to provide a sealable and sterilizable pouch which can withstand the pressures generated by steam sterilization at 125 °C and above and which has a large gas permeable area.

It is a further object of the invention to provide a sterilizable pouch which can be securely sealed relatively easily.

It is still a further object of the invention to provide a sealable and sterilizable pouch which can be opened easily.

### Summary of the Invention

The present invention provides a sealable sterilizable package suitable for sterilisation in the sealed state, comprising a first plastic sheet member securely heat sealed around a portion of its periphery to a second plastic sheet member to form a pocket with a heat sealable opening at one end to insert an object, said first plastic sheet member being substantially co-extensive with said second plastic sheet member, said first plastic sheet member including a third substantially gas impervious plastic sheet member and a membrane sheet member being sufficiently pervious to permit gas sterilisation but substantially impervious to bacteria, said membrane sheet member extending from a first position remote from said sealable opening to a second position closer to said sealable opening, said third plastic sheet member being securely heat sealed to said membrane sheet member adjacent to said second position, the sealable portion of said sealable opening being formed from said third sheet member and a substantially gas impervious portion of said second plastic sheet member, characterised in that:
the sealable sterilizable package is suitable for steam sterilisation at at least 125°C in the sealed state,
the membrane sheet member is sufficiently pervious to permit steam sterilisation at at least 125°C and
the second and third sheet members have at.least an outer layer of a thermoplastic polyolefine which can withstand steam sterilisation at at least 125°C for forming a heat seal of the sealable portion of said sealable opening.

The invention may provide a sealable sterilizable bag or pouch which can withstand steam sterilization at in excess of 125 °C without bursting.

The invention may also provide the advantage that the used bags are recyclable.

The invention may also provide a bag or pouch which is securely sealable and also relatively easy to open by tearing.

The invention may also provide a sterilizable bag with very high cleanliness.

The dependent claims define further embodiments of the present invention.

The invention, its embodiments and advantages will now be described with reference to the following drawings.

### Brief description of the drawings

Fig. 1A shows a schematic front view of a pouch in accordance with a first embodiment of the present invention.
Fig. 1B shows a schematic cross-section of the pouch shown in Fig. 1A.
Fig. 1C shows a detail of a cross-section of the pouch in accordance with Fig. 1A.
Fig. 2A shows a schematic front view of a pouch in accordance with a second embodiment of the present invention.
Fig. 2B shows a schematic cross-section of the pouch shown in Fig. 2A.
Fig. 2C shows a detail of a cross-section of the pouch in accordance with Fig. 2A.
Fig. 3A shows a schematic front view of a pouch in accordance with a third embodiment of the present invention.
Fig. 3B shows a schematic cross-section of the pouch shown in Fig. 3A.
Fig. 4A shows the sheet layout for a pouch in accordance with a fourth embodiment of the present invention.
Fig. 4B shows a schematic front view of a pouch in accordance with the fourth embodiment of the present invention.
Fig. 5A shows the sheet layout for a pouch in accordance with a fifth embodiment of the present invention.
Fig. 5B shows a schematic front view of a pouch in accordance with the fifth embodiment of the present invention.

### Description of the illustrative embodiments

The present invention will be described with reference to certain specific embodiments and to the drawings but the invention is not limited thereby but only by the claims. Further, in the drawings some of the dimensions, especially thicknesses of sheet materials may be exaggerated for clarity purposes.

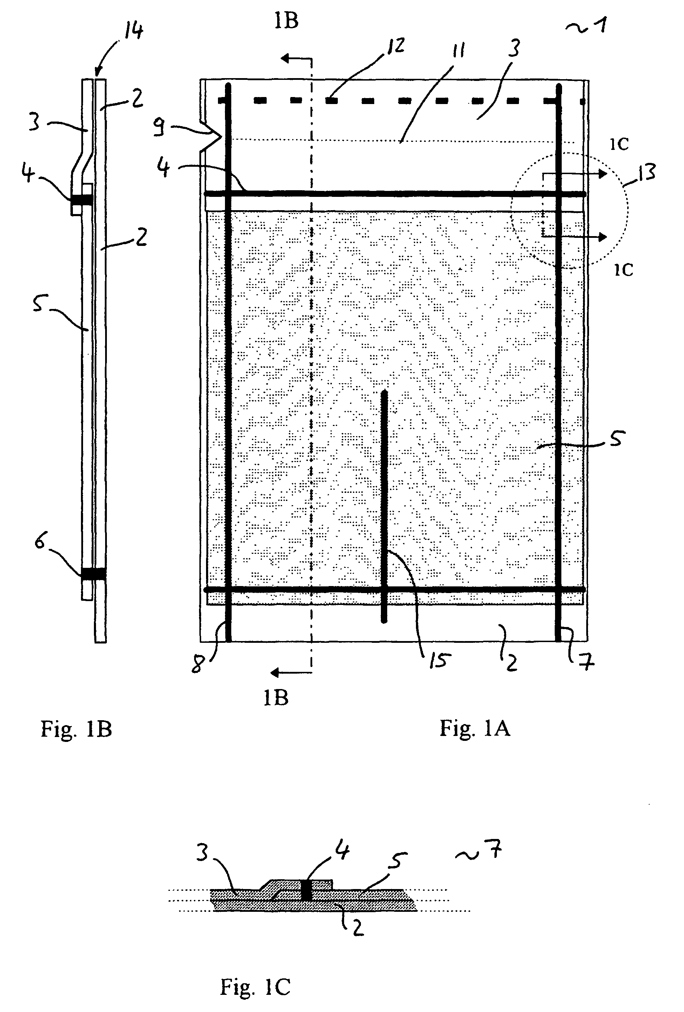

Fig. 1A shows schematically a sealable sterilizable package of bag 1 in accordance with a first embodiment of the present invention. Fig. 1B shows a cross section through bag 1. A substantially gas impervious plain plastic sheet material 3 is sealed to a membrane sheet 5 by means of a seal 4 which may be a heat seal or similar. Plain plastic sheet material 3 may be any suitable plastic material, preferably flexible, which can withstand steam sterilization of the closed and completely sealed bag 1 at at least 125 °C. It is particularly preferred if all the sheet materials used in the bags or pouches in accordance with the present invention are clean to at least class 200 and more preferably to class 100 or better in accordance with MIL-STD 1246C. It is also preferred if the plain sheet material 3 comprises at least two, preferably three or more discretely and separately formed layers which are intimately and permanently bonded together in order to reduce the risk of pinholes. At least an outer layer of plastic sheet material 3, which has to form a heat seal, may typically be a thermoplastic polyolefine which can withstand steam sterilization at at least 125°C, more preferably at at least 131°C. Non-limiting examples of suitable polyolefines are High Density Polyethylene (HDPE) or Polypropylene. High density polyethylene is a type of polyethylene made at low pressure by polymerising ethylene using catalysts. A suitable density range is 0.94 to 0.96 g/cm³, preferably 0.955 g/cm³. In particular, the sheet material 3 may be made from HDPE grade GM 9255F supplied by Hoechst AG, Germany, for example by sheet extrusion or casting or by lay flat tubing extrusion methods. Low density polyethylene, or ultra low density polythene sheet materials have a generally too low softening temperature to withstand steam sterilizing at 125 °C or more.

The various layers of sheet material 3 may differ from the heat-sealable outer layer and may include a metal layer such as aluminium, other high temperature polymeric materials such as polyester (e.g. polyethylene teraphthalate), or a layer of paper on which information may be easily printed. Further, it is preferred if sheet material 3 is highly oriented and has the same direction of orientation in each layer. The direction of orientation should be such that the sheet 3 may be torn relatively easily in a direction starting from a pre-formed weakness 9 in one or more edges, e.g. a V-cut or simple straight cut. Sheet material 3 may be 30 to 150 micron in thickness, preferably 70 to 90 micron.

Membrane sheet material 5 is a surgical grade membrane being impervious or substantially impervious to bacteria and similar infectious bodies but being pervious to sterilizing gases such as ethylene oxide and including steam. Preferably, membrane sheet 5 is a high-strength, tear-resistant material having a Gurley Hill air permeability between 5 and 75 seconds in accordance with ASTM D 726 and a low liquid permeability of at least 100 mm water head, preferably more than 170 mm in accordance with ISO 811. Membrane sheet material 5 is sealed to sheet material 3 by means of a seal 4. Preferably, membrane sheet material 5 should be sealable to sheet material 3 by heat sealing to form a secure seal without destruction of the specific properties of each material. In accordance with the present invention a secure seal has a seal strength of at least 1,5 N/mm, preferably greater than 2 N/mm in accordance with specification NFT 54-122. Typically, membrane sheet material 5 may include a spunbonded polyolefine fibre web, for example a melt-blown polypropylene fibre web and spunbonded fibre sheet laminated thereto as described in US 5 308 691. Membrane sheet material 5 may be spunbonded polyethylene under the trade name Tyvek, grade L1073B from E. I. DuPont de Nemours, France. Membrane sheet material 5 may be 75 to 300 micron, preferably 110 to 260 micron in thickness.

Composite sheet 3, 5 is securely sealed to a further bacteria impervious plastic sheet material 2, by means of seals 6, 7 and 8 which should be arranged close to the edges of sheets 3, 5 and 2, i.e. in the marginal area of sheets 3, 5 and 2. Seals 4, 6, 7 and 8 are preferably made with a heat sealing machine such as an Automatic Pouch Machine made by GN Packaging Equipment of Canada. Seals 4 and 6 are preferably made by current impulse heat sealing with a typical on/off time of 0.25/0.5 sec. and a typical pressure of 40 kPa. Seals 7 and 8 may be made by normal heat sealing at a temperature in the range 150 to 240 °C and typical pressure of 30 kPa. Preferably, sheets 2 and 3 and 5 are substantially co-extensive. Sheet material 2 may be similar or identical to plain sheet material 3 and is preferably relatively easily and securely sealable thereto, preferably by heat sealing. Sheet material 2 is preferably 40 to 150 micron, more preferably 70 to 90 micron thick. Sheet 2 is preferably oriented in the same direction as sheet 3. It is preferable if sheets 2, 3 and 5 are all made from the same basic material, e.g. polyethylene or polypropylene so that the bag 1 is recyclable after use.

The secure seals 4, 6, 7 and 8 are preferably arranged to form a pocket or pouch having three sealed sides and an opening 14 in the fourth side. An object may be introduced into the opening 14 and stored in the pouch. Objects may be medical instruments, medicines, surgical components as well as semiconductor or microelectronic devices. Opening 14 is bounded on two sides by sheets 2 and 3 which in this area are plain sheets made of similar or identical materials and which are preferably relatively easy to form into a secure seal. The width of sheet material 3 should be such that a secure seal 12 (indicated as dashed line), preferably a heat seal 12, may be formed between sheets 2 and 3 and across the width of opening 14 from seal 7 to seal 8 so as to completely seal off the bag. After sealing, the bag is placed in an autoclave and vacuum applied to evacuate the bag. Then steam is introduced at a temperature of 125 °C or above. During steam sterilization, the steam may enter and leave the pouch via the membrane sheet 5, thus sterilizing the object in the bag and the inside of the bag. After sterilization, the bag is again evacuated. The bag 1 may also be sterilzed other sterilizing gases such as ethylene oxide and/or with gamma radiation.

Further, sheet 3 is preferably wide enough to include a tear zone 11. To start the tear a weakness 9, such as a notch, slit or V-shaped is provided in the edges of both sheet materials 2, 3 on one or both sides of the bag 1. The tear propagates relatively easily along a line (indicated by the theoretical line 11 for purposes of clarity only) joining the sides of bag 2 as the sheet materials 2 and 3 are oriented in the cross-direction of the pouch, i.e. in the tear direction. At least in the region of the tear zone 11 and the seal 12, sheet 2 is preferably a plain plastic sheet material similar to sheet 3.

It is convenient to manufacture bag 1 by forming secure seals 4 and 6 at the same time. To do this, sheets 2, 3 and 5 are cut to the right size and laid together. To prevent sheet 5 adhering to sheet 2 underneath seal 4, a PTFE foil may be placed between sheets 5 and 2 at this position.

Seals 6, 7, and 8 and sheets 5 and 2 have been described as forming a rectangular pouch. The invention is not limited thereto. Sheets 2, 5 may be cut to any desired shape and the seals 6, 7 and 8 arranged close to the periphery of the shape, i.e. marginally. Further seals, as indicated by 15, may be provided to subdivide the bag 1 into further compartments as required. Further, one or more openings 14 may be located on the other sides of bag 1 as required.

A detail of the area 13 is shown in Fig. 1C. It can be seen that three layers come together and that layer 3 seals to layer 5 and to layer 2 and also, in the area of the overlap of sheets 3 and 5, the triangular gap at the end of layer 5 is adequately sealed. It is a surprising advantage of the present invention that such a construction can be reliably manufactured in plastic materials suitable for steam sterilization at at least 125 °C.

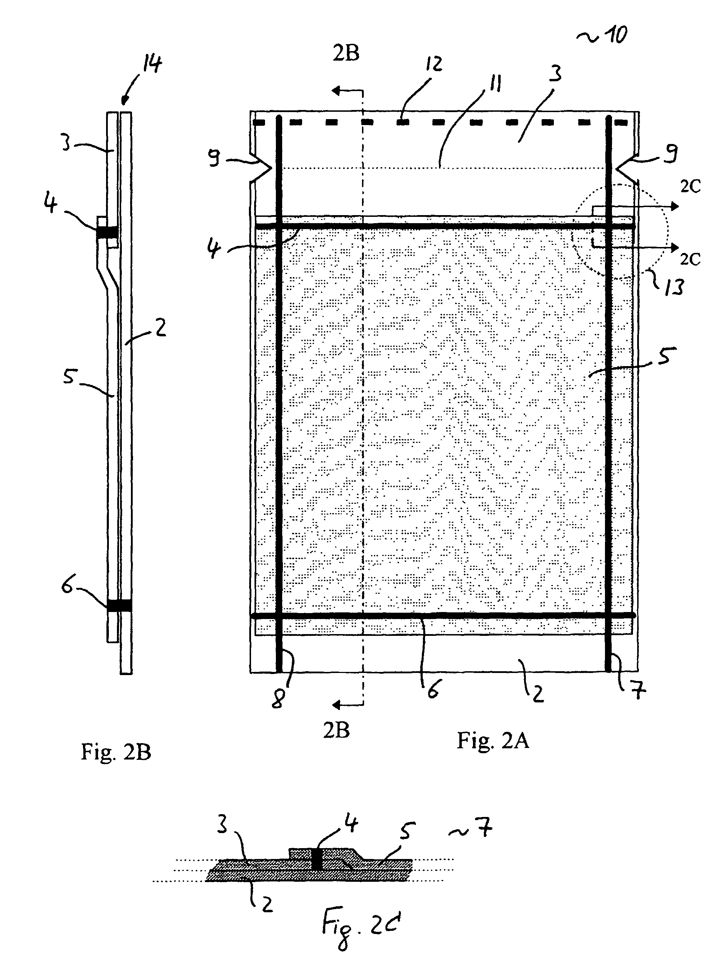

Fig. 2A shows a sealable sterilizable bag 10 in accordance with a second embodiment of the present invention. Fig. 2B show a schematic cross-section through the bag 10. Items with the same reference numbers as in the first embodiment may be made from materials identical to those described for the first embodiment. The major difference between embodiment 1 and 2 is that in embodiment 2, the plain plastic sheet material 3 is attached to sheet 5 in such a way that sheet 3 is closest to sheet 2 and sheet 5 is on the outside of seal 4. This arrangement is less preferred than embodiment 1 as it is normally easier to form a heat seal when the plastic sheet material 3 is on the outside.

As shown in the detail of region 13 in Fig. 2C, sheet 2 is securely sealed to sheet 3, sheet 5 is securely sealed to sheet 2 and in the area of overlap of sheets 3 and 5, the triangular gap at the end of sheet 3 is adequately sealed between sheet 5 and sheet 2. It is also a surprising advantage of this design, that a secure seal is obtained.

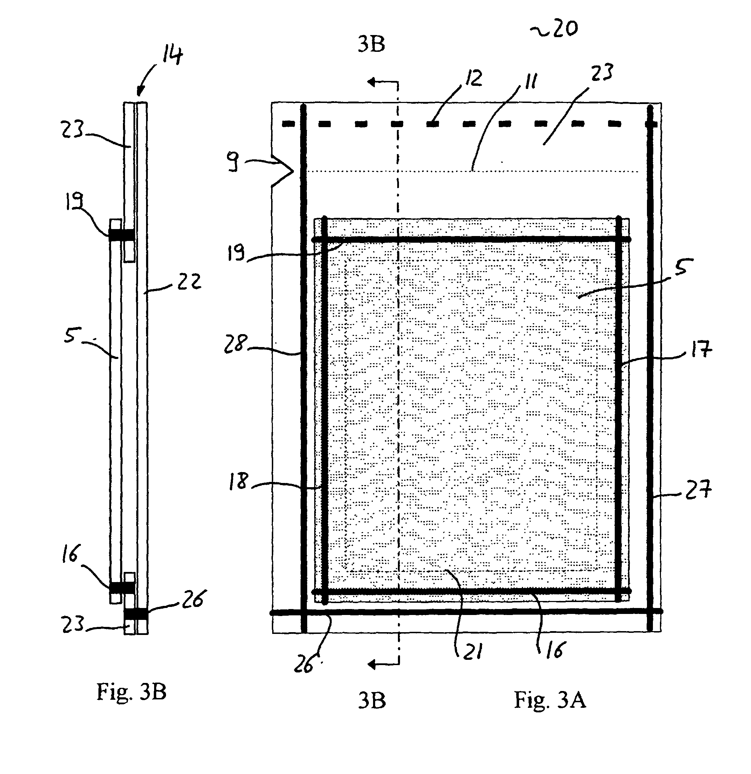

Fig. 3 A shows a schematic view of a sealable bag 20 in accordance with a third embodiment of the present invention. Fig. 3B shows a schematic cross-section through the bag 20. Items in the third embodiment with the same reference number are identical to those of the first and second embodiments.

An area 21 is cut out of a plain plastic sheet material 23, which may be similar or identical to sheet material 3 of the first and second embodiments. This area is covered by a membrane sheet 5 and securely sealed thereto by seals 16, 17, 18 and 19 to form a composite sheet 23, 5 which is substantially co-extensive with a further plastic sheet 22. Sheet material 22 may be similar or identical to sheet materials 2, 3 of the first and second embodiments. Sheet material 22 is securely sealed to sheet material 23 close to its periphery by seals 26, 27, 28 to form a pouch or pocket with a sealable opening 14. The secure seal 12, tear zone 11 and weakness 9 may be provided as described for the previous embodiments. At least in the region of secure seal 12, tear zone 11 and weakness 9, sheet 22 is preferably a plain plastic sheet material.

As an alternative, the membrane sheet 5 may securely sealed to the sheet material 23 around the cut-out area 21, but the sheet 5 is placed on the inside (not shown) when composite sheet 23, 5 is securely sealed to sheet material 23.

The third embodiment avoids the triple-layer seal in region 13 of the first and second embodiments but has the disadvantage of three extra seals and hence a reduced volume for the pouch or bag 20 for comparable outer dimensions.

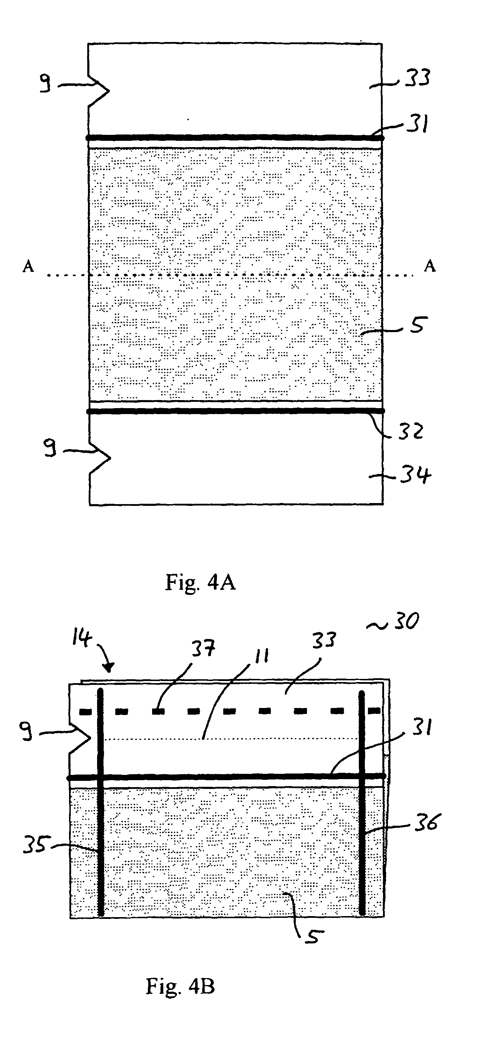

Fig. 4B shows a schematic representation of a fourth embodiment of a sealable sterilizable pouch 30 in accordance with the present invention. Fig. 4A shows the layout of the relevant sheet materials before finishing. Plain sheet materials 33 and 34 are securely sealed to a piece of membrane sheet 5. Plain sheet materials 33 and 34 may be any of the materials mentioned with respect to sheet materials 2, 3 for the first and second embodiments and sheet 5 is any of the materials mentioned with respect to membrane sheet 5 of the first and second embodiments. Seals 31 and 32 are preferably secure heat seals. Notches 9 may also be introduced at this time. The composite sheet 33, 34, 5 is folded along line A - A and the side edges closed with seals 35 and 36 to form a pouch 30 with an opening 14 at the top. Seals 35 and 36 are preferably secure heat seals. To close the pouch 30 a seal 37 may be made as indicated by the dotted line 37. The seal is preferably a secure heat seal. The pouch 30 may be opened by tearing from the notch 9 along a line indicated by 11.

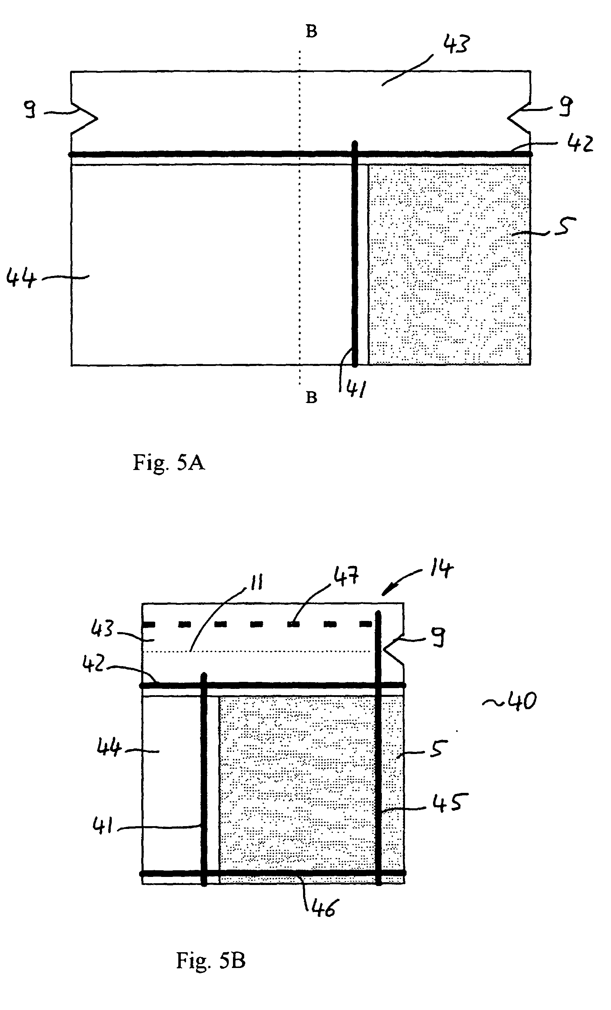

Fig. 5B shows a schematic representation of a fifth embodiment of a sealable sterilizable pouch 40 in accordance with the present invention. Fig. 5A shows the layout of the relevant sheet materials before finishing. Plain sheet materials 43 and 44 are securely sealed to a piece of membrane sheet 5. Plain sheet materials 43 and 44 may be any of the materials mentioned with respect to sheet materials 2, 3 for the first and second embodiments and 5 is any of the materials mentioned with respect to membrane sheet 5 of the first and second embodiments. Sheet materials 43, 44 may be a single piece of sheet with a section cut out for the membrane sheet 5. Seals 41 and 42 are preferably secure heat seals. Notches 9 may also be introduced at this time. The composite sheet 43, 44, 5 is folded along line B - B and the side edge closed with a secure seal 45 and the bottom edge with a secure seal 46 to form a pouch 40 with an opening 14 at the top. Seals 45 and 46 are preferably secure heat seals. To close the pouch 40 a secure seal 47 may be made as indicated by the dotted line 47. The seal is preferably a secure heat seal. The pouch 40 may be opened by tearing from the notch 9 along a theoretical line indicated by 11.

In accordance with all the embodiments of the present invention, the sheet materials forming the lips of the opening 14 are preferably made from plain sheet materials which are easily sealed in a secure manner.

In accordance with all the embodiments of the present invention, it is preferable if the area of the membrane sheet material 5 is at least 50%, more preferably at least 80% of the area of one side of the bag 1, 10, 20, 30, 40 in order to provide a low resistance to steam and/or gas flow in and out of the bag 1, 10, 20, 30, 40.

In all the embodiments of the present invention, characters or graphics may be printed on any part of the bag 1, 10, 20, 30, 40. For instance, sterilization indicators, tear zone or seal zone indicators may be printed in suitable positions.

## Claims

1. Sealable sterilizable package (1) suitable for sterilisation in the sealed state, comprising a first plastic sheet member (3, 5) securely heat sealed around a portion of its periphery to a second plastic sheet member (2) to form a pocket with a heat sealable opening (14) at one end to insert an object, said first plastic sheet member (3, 5) being substantially co-extensive with said second plastic sheet member (2), said first plastic sheet member (3, 5) including a third substantially gas impervious plastic sheet member (3) and a membrane sheet member (5) being sufficiently porous to permit gas sterilisation but substantially impervious to bacteria, said membrane sheet member (5) extending from a first position remote from said sealable opening (14) to a second position closer to said sealable opening (14), said third plastic sheet member (3) being securely heat sealed to said membrane sheet member (5) adjacent to said second position, the sealable portion of said sealable opening (14) being formed from said third sheet member (3) and a substantially gas impervious portion of said second plastic sheet member (2), **characterised in that**:
the sealable sterilizable package is suitable for steam sterilisation at at least 125°C in the sealed state,
the membrane sheet member (5) is sufficiently pervious to permit steam sterilisation at at least 125°C and
the second and third sheet members (2, 3) have at least an outer layer of a thermoplastic polyolefine which can withstand steam sterilisation at at least 125°C for forming a heat seal of the sealable portion of said sealable opening (14).

2. Sealable sterilizable package according to claim 1, **characterised in that** the second (2) and/or third (3) sheet members are laminates.

3. Sealable sterilizable package according to claim 1 or 2 **characterised in that** said second plastic sheet member (2) comprises one of polypropylene and high density polyethylene.

4. Sealable sterilizable package according to any of the preceding claims further **characterised by** a tear promoter (9) located in the edge regions of said third sheet member (3) and the substantially gas impervious portion of said second plastic sheet member (2).

5. Sealable sterilizable package according to claim 4, **characterised in that** said third sheet member (3) and the substantially gas impervious portion of said second plastic sheet member (2) are both oriented and the direction of orientation is substantially the same as the direction of tear starting at said tear promoter (9).

6. Sealable sterilizable package according to any of the preceding claims, **characterised in that** the secure seals (4, 6) have a tear strength of at least 1.5 N/mm, more preferably at least 2N/mm.

7. Sealable sterilizable package according to any of the preceding claims **characterised in that** said third sheet member (3) extends across the package (1) from a first side to a second side and one edge of said third sheet member (3) defines one lip of said opening, an edge of said second plastic sheet member (2) defines the other lip of said opening (14) and a further edge of said third sheet member (3) remote from said opening (14) is securely sealed to said membrane sheet member (5).

8. Sealable sterilizable package according to claim 7 **characterised in that** the membrane sheet (5) lies between the third sheet member (3) and the second sheet member (2) at the seal position of the third sheet member (3) and the membrane (5) sheet member.

9. Sealable sterilizable package according to any of the preceding claims which is recyclable.

10. A sealed package (1) made from a sealable sterilizable package according to any of the previous claims.

11. A sterilisation method including the use of a sealed package (1) according to claim 10.

12. A sterilisation method according to claim 11 which includes the use of at least one of steam, ethylene oxide or gamma radiation sterilization.

13. Sealable sterilizable package according to any of claims 1-9, wherein the membrane sheet member (5) has a Gurley Hill air permeability between 5 and 75 seconds, as determined in accordance with ASTM D 726.

14. Sealable sterilizable package according to claim 13, wherein membrane sheet member (5) further has a liquid permeability of at least 100 mm water head, as determined in accordance with ISO 811.

15. Sealable sterilizable package according to any of claims 1-9, 13 or 14, wherein the membrane sheet member (5) comprises a melt-blown polypropylene fiber web and spun bonded fiber sheet laminated thereto.

16. Sealable sterilizable package according to any of claims 1-9 or 13-15, wherein the membrane sheet member (5) has a thickness of 75 to 300 microns.

17. Sealable sterilizable package according to any of claims 1-9 or 13-16, wherein the membrane sheet member (5) is securely sealed in a cut-out area of one of the first and second plastic sheet members.

18. Sealable sterilizable package according to any of claims 1-9 or 13-17, wherein the membrane sheet member (5) has an area that is at least 50% of the area of one side of the bag defined by one of said first and second plastic sheet members.

19. Sealable sterilizable package according to any of claims 1-9 or 13-17, wherein the membrane sheet member (5) has an area that is at least 80% of the area of one side of the bag defined by one of said first and second plastic sheet members.

## Patentansprüche

1. Versiegelbare, sterilisierbare Verpackung (1), geeignet zur Sterilisierung im verschlossenen Zustand, umfassend ein erstes Plastikfolienelement (3, 5), das um einen Teil seines Rands mit einem zweiten Plastikfolienelement (2) sicher wärmeversiegelt ist, um eine Tasche zu bilden, die an einem Ende eine wärmeversiegelbare Öffnung (14) zum Einführen eines Objekts aufweist, wobei das erste Plastikfolienelement (3, 5) mit dem zweiten Plastikfolienelement (2) im Wesentlichen flächengleich ist, wobei das erste Plastikfolienelement (3, 5) ein drittes im Wesentlichen gasundurchlässiges Plastikfolienelement (3) und ein Membranfolienelement (5) aufweist, das porös genug ist, um eine Gassterilisierung zu erlauben, für Bakterien aber im Wesentlichen undurchlässig ist, wobei das Membranfolienelement (5) sich von einer ersten Position, die von der versiegelbaren Öffnung (14) entfernt ist, zu einer zweiten Position erstreckt, die der versiegelbaren Öffnung (14) näher liegt, wobei das dritte Plastikfolienelement (3) in der Nähe der zweiten Position mit dem Membranfolienelement (5) wärmeversiegelt ist, wobei der versiegelbare Teil der versiegelbaren Öffnung (14) aus dem dritten Folienelement (3) und einem im Wesentlichen gasundurchlässigen Teil des zweiten Plastikfolienelements (2) gebildet ist, **dadurch gekennzeichnet, dass**:
- die versiegelbare, sterilisierbare Verpackung im versiegelten Zustand zur Dampfsterilisierung bei mindestens 125°C geeignet ist,
- das Membranfolienelement (5) genügend durchlässig ist, um eine Dampfsterilisierung bei mindestens 125°C zu erlauben, und
- das zweite und das dritte Folienelement (2, 3) mindestens eine äußere Schicht aus einem thermoplastischen Polyolefin haben, das einer Dampfsterilisierung bei mindestens 125°C standhält, um eine Wärmeversiegelung des versiegelbaren Teils der versiegelbaren Öffnung (14) zu bilden.

2. Versiegelbare, sterilisierbare Verpackung nach Anspruch 1, **dadurch gekennzeichnet, dass** das zweite (2) und/oder das dritte (3) Folienelement Schichtstoffe sind.

3. Versiegelbare, sterilisierbare Verpackung nach Anspruch 1 oder 2, **dadurch gekennzeichnet, dass** das zweite Plastikfolienelement (2) entweder ein Polypropylen oder ein hochdichtes Polyethylen umfasst.

4. Versiegelbare, sterilisierbare Verpackung nach einem der vorhergehenden Ansprüche, **gekennzeichnet durch** eine Sollrissstelle (9), die in den Kantenbereichen des dritten Folienelements (3) und des im Wesentlichen gasundurchlässigen Teils des zweiten Plastikfolienelements (2) angeordnet ist.

5. Versiegelbare, sterilisierbare Verpackung nach Anspruch 4, **dadurch gekennzeichnet, dass** sowohl das dritte Folienelement (3) als auch der im Wesentlichen gasundurchlässige Teil des zweiten Plastikfolienelements (2) orientiert sind und die Orientierungsrichtung im Wesentlichen die gleiche wie die Richtung des Risses ist, der an der Sollrissstelle (9) beginnt.

6. Versiegelbare, sterilisierbare Verpackung nach einem der vorhergehenden Ansprüche, **dadurch gekennzeichnet, dass** die sicheren Versiegelungen (4, 6) eine Reißfestigkeit von mindestens 1,5 N/mm und vorzugsweise von 2 N/mm haben.

7. Versiegelbare, sterilisierbare Verpackung nach einem der vorhergehenden Ansprüche, **dadurch gekennzeichnet, dass** sich das dritte Folienelement (3) über die Verpackung (1) von einer ersten Seite zu einer zweiten Seite erstreckt und eine Kante des dritten Folienelements (3) eine Lippe der Öffnung definiert, eine Kante des zweiten Plastikfolienelements (2) die andere Lippe der Öffnung (14) definiert und eine weitere Kante des dritten Folienelements (3), die von der Öffnung (14) entfernt ist, mit dem Membranfolienelement (5) sicher versiegelt ist.

8. Versiegelbare, sterilisierbare Verpackung nach Anspruch 7, **dadurch gekennzeichnet, dass** die Membranfolie (5) an der Versiegelungsposition des dritten Folienelements (3) und des Membranfolienelements (5) zwischen dem dritten Folienelement (3) und dem zweiten Folienelement (2) angeordnet ist.

9. Versiegelbare, sterilisierbare Verpackung nach einem der vorhergehenden Ansprüche, die recyclingfähig ist.

10. Versiegelte Verpackung (1), hergestellt aus einer versiegelbaren, sterilisierbaren Verpackung nach einem der vorhergehenden Ansprüche.

11. Sterilisierungsverfahren, das die Verwendung einer versiegelten Verpackung (1) nach Anspruch 10 beinhaltet.

12. Sterilisierungsverfahren nach Anspruch 11, das die Verwendung von mindestens einer Dampf-, einer Ethylenoxid- oder einer Gammastrahlensterilisierung beinhaltet.

13. Versiegelbare, sterilisierbare Verpackung nach einem der Ansprüche 1-9, bei der das Membranfolienelement (5) eine nach ASTM D 726 bestimmte Gurley-Hill-Luftdurchlässigkeit von zwischen 5 und 75 Sekunden hat.

14. Versiegelbare, sterilisierbare Verpackung nach Anspruch 13, bei der das Membranfolienelement (5) weiter eine nach ISO 811 bestimmte Flüssigkeitspermeabilität von mindestens 100 mm Wassersäule hat.

15. Versiegelbare, sterilisierbare Verpackung nach einem der Ansprüche 1-9, 13 oder 14, bei der das Membranfolienelemente (5) eine schmelzgeblasene Polypropylenfaserfolienbahn und eine darauf laminierte spinngebundene Faserfolie umfasst.

16. Versiegelbare, sterilisierbare Verpackung nach einem der Ansprüche 1-9 oder 13-15, bei der das Membranfolienelement (5) eine Dicke zwischen 75 und 300 Mikrometer hat

17. Versiegelbare, sterilisierbare Verpackung nach einem der Ansprüche 1-9 oder 13-16, bei der das Membranfolienelement (5) sicher mit einem ausgeschnittenen Bereich entweder des ersten oder des zweiten Plastikfolienelements versiegelt ist.

18. Versiegelbare, sterilisierbare Verpackung nach einem der Ansprüche 1-9 oder 13-17, bei der das Membranfolienelement (5) eine Fläche hat, die mindestens 50% der Fläche einer Seite der Verpackung ist, die durch entweder das erste oder das zweite Plastikfolienelement gebildet wird.

19. Versiegelbare, sterilisierbare Verpackung nach einem der Ansprüche 1-9 oder 13-17, bei der das Membranfolienelement (5) eine Fläche hat, die mindestens 80% der Fläche einer Seite der Verpackung ist, die durch entweder das erste oder das zweite Plastikfolienelement gebildet wird.

## Revendications

1. Emballage pouvant être stérilisé et pouvant être scellé (1) approprié pour une stérilisation dans l'état scellé, comprenant un premier élément formant feuille en plastique (3, 5) solidement scellé à la chaleur autour d'une partie de sa périphérie à un deuxième élément formant feuille en plastique (2) pour former une poche avec une ouverture pouvant être scellée à la chaleur (14) au niveau d'une extrémité pour insérer un objet, ledit premier élément formant feuille en plastique (3, 5) étant sensiblement coextensif avec ledit deuxième élément formant feuille en plastique (2), ledit premier élément formant feuille en plastique (3, 5) comprenant un troisième élément formant feuille en plastique sensiblement imperméable aux gaz (3) et un élément formant feuille de membrane (5) suffisamment poreux pour permettre une stérilisation au gaz, mais sensiblement imperméable aux bactéries, ledit élément formant feuille de membrane (5) s'étendant depuis une première position éloignée de ladite ouverture pouvant être scellée (14) jusqu'à une seconde position tout près de ladite ouverture pouvant être scellée (14), ledit troisième élément formant feuille en plastique (3) étant solidement scellé à la chaleur audit élément formant feuille de membrane (5) adjacent à ladite seconde position, la partie pouvant être scellée de ladite ouverture pouvant être scellée (14) étant formée à partir dudit troisième élément formant feuille (3) et d'une partie sensiblement imperméable aux gaz dudit deuxième élément formant feuille en plastique (2), **caractérisé en ce que** :
l'emballage pouvant être stérilisé et pouvant être scellé est approprié pour une stérilisation à la vapeur à au moins 125 °C dans l'état scellé,
l'élément formant feuille de membrane (5) est suffisamment perméable pour permettre une stérilisation à la vapeur à au moins 125 °C, et
les deuxième et troisième éléments formant feuilles (2, 3) ont au moins une couche extérieure en polyoléfine thermoplastique qui peut résister à une stérilisation à la vapeur à au moins 125 °C pour former un joint à la chaleur de la partie pouvant être scellée de ladite ouverture pouvant être scellée (14).

2. Emballage pouvant être stérilisé et pouvant être scellé selon la revendication 1, **caractérisé en ce que** les deuxième (2) et/ou troisième (3) éléments formant feuilles sont des stratifiés.

3. Emballage pouvant être stérilisé et pouvant être scellé selon la revendication 1 ou 2, **caractérisé en ce que** ledit deuxième élément formant feuille en plastique (2) est constitué par l'un du polypropylène et du polyéthylène de densité élevée.

4. Emballage pouvant être stérilisé et pouvant être scellé selon l'une quelconque des revendications précédentes, **caractérisé de plus par** une amorce de déchirure (9) placée dans les zones de bord dudit troisième élément formant feuille (3) et de la partie sensiblement imperméable aux gaz dudit deuxième élément formant feuille en plastique (2).

5. Emballage pouvant être stérilisé et pouvant être scellé selon la revendication 4, **caractérisé en ce que** ledit troisième élément formant feuille (3) et la partie sensiblement imperméable aux gaz dudit deuxième élément formant feuille en plastique (2) sont tous les deux orientés et le sens d'orientation est sensiblement le même que le sens de démarrage de la déchirure au niveau de ladite amorce de déchirure (9).

6. Emballage pouvant être stérilisé et pouvant être scellé selon l'une quelconque des revendications précédentes, **caractérisé en ce que** les joints sûrs (4, 6) ont une résistance à la déchirure d'au moins 1,5 N/mm, encore mieux d'au moins 2 N/mm.

7. Emballage pouvant être stérilisé et pouvant être scellé selon l'une quelconque des revendications précédentes, **caractérisé en ce que** ledit troisième élément formant feuille (3) s'étend d'un bout à l'autre de l'emballage (1) depuis un premier côté jusqu'à un second côté et un bord dudit troisième élément formant feuille (3) définit un rebord de ladite ouverture, un bord dudit deuxième élément formant feuille en plastique (2) définit l'autre rebord de ladite ouverture (14) et un bord supplémentaire dudit troisième élément formant feuille (3) éloigné de ladite ouverture (14) est solidement scellé audit élément formant feuille de membrane (5).

8. Emballage pouvant être stérilisé et pouvant être scellé selon la revendication 7, **caractérisé en ce que** la feuille de membrane (5) se situe entre le troisième élément formant feuille (3) et le deuxième élément formant feuille (2) à la position de joint du troisième élément formant feuille (3) et de l'élément formant feuille de membrane (5).

9. Emballage pouvant être stérilisé et pouvant être scellé selon l'une quelconque des revendications précédentes qui est recyclable.

10. Emballage scellé (1) réalisé à partir d'un emballage pouvant être stérilisé et pouvant être scellé selon l'une quelconque des revendications précédentes.

11. Procédé de stérilisation comprenant l'utilisation d'un emballage scellé (1) selon la revendication 10.

12. Procédé de stérilisation selon la revendication 11 qui comprend l'utilisation d'au moins une entre la stérilisation à la vapeur, à l'oxyde d'éthylène ou aux rayons gamma.

13. Emballage pouvant être stérilisé et pouvant être scellé selon l'une quelconque des revendications 1 à 9, dans lequel l'élément formant feuille de membrane (5) a une perméabilité Gurley Hill à l'air comprise entre 5 et 75 secondes, comme on le détermine selon ASTM D 726.

14. Emballage pouvant être stérilisé et pouvant être scellé selon la revendication 13, dans lequel l'élément formant feuille de membrane (5) a de plus une perméabilité aux liquides d'au moins 100 mm de hauteur d'eau, comme on le détermine selon ISO 811.

15. Emballage pouvant être stérilisé et pouvant être scellé selon l'une quelconque des revendications 1 à 9, 13 ou 14, dans lequel l'élément formant feuille de membrane (5) comprend un tissu de fibres de polypropylène soufflées et fondues et d'une feuille de fibres liées et filées stratifiée à ce dernier.

16. Emballage pouvant être stérilisé et pouvant être scellé selon l'une quelconque des revendications 1 à 9 ou 13 à 15, dans lequel l'élément formant feuille de membrane (5) a une épaisseur de 75 à 300 microns.

17. Emballage pouvant être stérilisé et pouvant être scellé selon l'une quelconque des revendications 1 à 9 ou 13 à 16, dans lequel l'élément formant feuille de membrane (5) est solidement scellé dans une zone de découpe de l'un des premier et deuxième éléments formant feuilles en plastique.

18. Emballage pouvant être stérilisé et pouvant être scellé selon l'une quelconque des revendications 1 à 9 ou 13 à 17, dans lequel l'élément formant feuille de membrane (5) a une surface qui fait au moins 50 % de la surface d'un côté du sachet défini par l'un desdits premier et deuxième éléments formant feuilles en plastique.

19. Emballage pouvant être stérilisé et pouvant être scellé selon l'une quelconque des revendications 1 à 9 ou 13 à 17, dans lequel l'élément formant feuille de membrane (5) a une surface qui fait au moins 80 % de la surface d'un côté du sachet défini par l'un desdits premier et deuxième éléments formant feuilles en plastique.
